# EUROPEAN PATENT SPECIFICATION

(11) **EP 3 185 825 B1**
(45) Date of publication and mention of the grant of the patent: **15.12.2021**
(21) Application number: 15836170.9
(22) Date of filing: 21.08.2015
(51) Int. Cl.: A61F 9/04

(54) **SURGICAL EYE SHIELD**
CHIRURGISCHER AUGENSCHUTZ
ÉCRAN CHIRURGICAL DE PROTECTION DES YEUX

(30) Priority: 29.08.2014 AU 2014903456
(43) Date of publication of application: 05.07.2017
(73) Proprietor: Innovgas Pty Ltd, Launceston, Tasmania 7250 (AU)
(72) Inventor: WALLIS, Andrew, East Launceston, Tasmania 7250 (AU)
(74) Representative: O'Connor, Dominic David
(86) International application number: PCT/AU2015/050481
(87) International publication number: WO 2016/029257

(56) References cited:
- EP-A1- 0 516 169
- US-A- 2 359 743
- US-A- 2 603 785
- US-A- 4 122 847
- US-A- 4 649 908
- US-A- 6 098 628
- US-A- 6 155 261
- US-A1- 2005 022 823
- US-A1- 2012 192 330
- US-A1- 2014 251 342
- US-B1- 7 748 387
- None

## Description

### FIELD OF THE INVENTION

The present invention relates to a perioperative eye shield for inhibiting pressure being applied to the eyes of a patient during a medical procedure.

### BACKGROUND OF THE INVENTION

During a medical procedure the face of a sedated patient is often covered by surgical drapes. The eye region of the patient is therefore obscured by these drapes.

If the medical procedure is being undertaken in or on the upper half of the body there is a considerable risk that a surgeon, anaesthetist, nurse or other medical practitioner may lean on or place instruments on the face and inadvertently the eye region of the patient. This is particularly the case with ear, nose and throat surgery (ENT), cardiothoracic surgery, dental surgery, some plastic surgery and reconstructive surgery, some orthopaedic surgery, as well as some general surgery.

Accordingly, if direct pressure is inadvertently applied to the patient's eye or eyes, trauma can occur. This may be temporary loss or reduction of vision, bruising or may result in eye globe trauma leading to transient or permanent damage.

Currently many anaesthetists simply tape eye pads across the eye sockets. This however means that the eyelids are not visible during the medical procedure and may open without the knowledge of the medical practitioner. The opening of the eyes may lead to injury or irritation of the patient's eye by corneal drying or abrasion. Furthermore the eye pads do not prevent direct pressure being applied to the eye globes but simply reduces the degree of force, which may still be insufficient to avoid eye trauma.

There are eye guards disclosed in the prior art that include a mask, which is secured to a person's face by a strap that extends around the back of the patient's head. There are however a number of problems with such designs. Firstly, the strap has to be tight enough to hold the mask in place during the medical procedure and therefore the strap can cut into the ears and head of the patient. Furthermore, the use of a strap is problematic in that it can be easily caught or displaced, especially when a patient is being rolled onto their side, as occurs in some medical procedures.

There are also a number of eye guards disclosed in the published prior art that are secured using an adhesive backing rather than a strap. These include United States Patent No. 4,122,847 to CRAIG, which discloses an eye shield for surgical patients that includes bulbous eye covers. These moulded eye covers however protrude excessively outwardly from the face of the patient, which increases the likelihood that the shield would be bumped during the medical procedure. Furthermore, the edges of the moulded eye covers are curved inwardly towards the edges of the eye sockets, which means they may impinge upon the eye when pressure is applied to the front of the cover. Another limitation with the eye cover disclosed in CRAIG is that since the distance between the bulbous eye covers is fixed, and the bulbous eye covers must be positioned directly over respective eyes of the patient, there is only a very limited range of face sizes that the eye guard will accurately fit. This inability of the face guard of CRAIG to fit multiple face sizes and shapes, is a significant problem.

Another guard disclosed in the prior art, is found in United States Patent 6,098,628 to FUNK, which teaches separate moulded eye guards that create respective chambers to protect the eye from direct contact. The two eye guards are attached by way of a bridge member that provides an adjustable fit for the size and contours of different faces. The eye guard disclosed in FUNK have a similar configuration to a pair of swimming goggles and would therefore still impinge upon the edges of the eye if direct pressure were applied. Furthermore, the separate eye guards would interfere with eye patches used to hold the eyelids closed during surgery. Documents US-A-4,122,847, US-A-4,649,908, US-A-2012/0192330, US-B-7,748,387 disclose the preamble of claim 1.

### SUMMARY OF THE INVENTION

According to an aspect of the present invention, there is provided an eye shield as claimed in claim 1.

The eye shield in one form is a perioperative eye shield. The eye shield may be a single use perioperative apparatus that covers both eyes of the patient to inhibit pressure being applied to the eyes.

Preferably the angular displacement between the first and second eye cover portions produced by the hinge member is greater than the angular displacement produced across each respective first and second eye cover portions as they flex along their span. The term span is to be giving its ordinary means: full extent of something from end to end.

In a preferred form the first and second eye cover portions flex along their entire respective spans, however it should be appreciate that a part or parts of the first and second eye cover portions may not flex to provide greater rigidity in particular areas.

The degree of flexing at different locations across the span of each eye cover portion may vary or may be uniform. The edges of the eye cover portion may flex to a greater degree than a portion that is adjacent the hinge member.

The hinge member in one form comprises a plurality of holes that extend through at least the cover plate at a mid region that corresponds to the position of a bridge of the patient's nose when the eye shield is attached to the face of said patient.

The holes that form the hinge member may be similar size and shape or may be dissimilar in size and shape.

In another form holes extend through the resiliently deformable base and coaxially align the holes through the cover plate and are of similar size and shape.

Alternatively, the transparent cover plate may be constructed from two individual eye cover portions that are fixedly joined along adjacent edges by the hinge member, said adjacent edges parallel and overlaying the bridge of the patient's nose when the eye shield is attached to the face of said patient.

The transparent cover plate is curved and may have a uniform thickness or may have varied thickness thereacross. The cover plate can be bent or flexed thereacross along the width and height of each of the first and second eye cover portions.

Preferably the cover plate is curved along a line extending longitudinally across the eye shield and is also curved along a line extending laterally down the eye shield. In this way the cover plate and resiliently deformable base attached thereto are curved generally in both vertical and horizontal directions. This ensures that the eye shield conforms, to a degree, to the sides or temples and cheek regions of the patient's face. The curvature means that the tendency of the cover plate to pull away from the face, which could peel the base off the skin, is minimised in the areas on the face to which it is attached that have the most abrupt contours.

The eye shield is configured to conform closely to the face of the patient and not project excessively outwardly from the face of the patient. This low profile means that the eye shield is less likely to get in the way of a surgeon during a medical procedure. Accordingly in one form each of the first and second eye cover portions are generally curved rearwardly along a longitudinal axis extending outwardly from the hinge member. This means that the outer portion of each eye cover portion, at an end opposite the hinge member is curved towards the face of the patient and therefore assists in it's attachment to the temples of the patient.

Furthermore, the lower portion of each of the first and second eye cover portions may be curved rearwardly to assist in the attachment to respective cheeks of the patient.

The transparent cover plate extends beyond the inner parts of the orbital rims such that when pressure is applied to a front of the transparent cover plate the force is distributed around the bony orbital rims and bridge of the patient's nose, wherein the eye shield is inhibited from impinging upon an eye or eyelids of said patient. The reader will appreciate that when pressure is applied to one side or part of the eye shield the force is distributed across the entire orbital rim or rims due to the unitary construction of the cover plate. The transparent cover plate in one form is a generally thin piece of material having uniform thickness, however it should be appreciate that the cover plate can vary in thickness. The transparent cover plate is semi-rigid to inhibit the eye shield from bowing inwardly and thereby coming into contact with the eye of the patient. However the transparent cover plate is still flexible enough to permit bending of the eye shield around the contours of the face.

The transparent cover plate may be constructed from a clear plastic material, including polyethylene, polyvinylidene, polypropylene, or any other type of material either synthetic or natural, that provide a flexible transparent cover plate.

The transparent cover plate is pre-curved or moulded and is flexible enough to further conform to the curvature of face when applied. In an example, not part of the invention, the transparent cover plate is generally planar and is able to be flexed around the contours of the patient's face.

Preferably the transparent cover plate is constructed from a single piece or two joined portions that form a single member to cover both eyes. This means that when pressure is applied to one side of the eye shield the force is distributed across both sides of the face and the bridge of the nose. This is important since it inhibits the edges of the cover plate from cutting into the edges of the eye as would occur at adjacent sides of the nose with the devices suggested in the published prior art.

The transparent cover plate is strong enough to resist pressure being routinely applied to it in a medical situation. The transparent cover plate may also include ridges to increase the rigidity of the eye shield. In one form the ridges are generally perpendicular to the longitudinal axis of the eye shield. The transparent cover plate may also include grooves that extend down a front or rear, which permit greater flexing of the transparent cover plate, especially adjacent the temples of the patient.

The resiliently deformable base includes two spaced apart apertures extending therethrough that can be positioned by a user to correspond to the location of eye sockets of the patient. The resiliently deformable base further includes an adhesive central part that can be positioned to correspond to the location of the bridge of the patient's nose, to thereby create respective chambers to protect the eyes from direct contact, when the eye shield is attached to the face of the patient. By resiliently deformable the reader should understand that the base may be deformed, but has a tendency to expand back to, or close to, its original shape. This so called 'memory' of the material means that it tends to regain its original shape when not under pressure, and therefore is useful in conforming to the contoured surface around the eyes of the patient.

The resiliently deformable base in one form is a medium density polyurethane foam, such that it is able to conform to the face of a patient. The resiliently deformable base is relatively thick in comparison to the transparent cover plate.

The resiliently deformable base may be constructed from a soft, flexible, low-density, open-cell, thermoplastic foam material. The low-density foam, may have a density of around 0.1 g/cm³ or less, a stiffness of around 600 milligrams or less, and an edge compression of between 220-260 grams or less, although other densities are possible within the scope of the invention. It should be appreciated that the resiliently deformable base is not limited to the above low-density foam.

In another form the resiliently deformable base may be constructed from viscoelastic polyurethane foam that reacts to body heat, allowing it to mould to a warm body.

In one form the resiliently deformable base has outer dimensions that are slightly larger than the transparent cover plate to prevent the edges of cover plate touching the patient when external pressure is applied, which could otherwise cause it to cut into the face of the patient.

In one form a peelable protective film extends over the adhesive layer of said base, which protects the adhesive coating prior to use. The peelable protective film can be removed prior to the eye shield being attached over the orbital rims of the patient.

The resiliently deformable base may have a uniform thickness of between 10-12 mm. Alternatively, the base may include some moulding to facilitate attachment to the face. In one form the central part of the base that is configured for attachment to the nose of the patient may be curved around a longitudinal axis to conform to the bridge of the nose. Similarly the outmost edges of the base may be thicker to facilitate attachment to temples of the patient.

The base may include a non-adhesive tab or portion on at least one side of the shield to aid in the application and/or removal of the eye shield. In one form the eye shield includes two non-peelable tabs, one at each side of the base, each having a respective non-adhesive outer surface. Although it should be appreciated that the non-adhesive tab or portion is not essential and the practitioner can simply grasp an edge of the base and peel it off the patient's skin to initiate the removal of the eye shield from the patient's face.

The transparent cover plate may include vent holes extending therethrough to inhibit condensation forming within the respective chambers.

The eye shield preferably permits the attachment of eye dressings or tape that are attached over the closed eyelids of a patient to maintain the eyelids closed during surgery to inhibit corneal drying or abrasion. Furthermore, since the cover plate is transparent and the base includes apertures, the medical practitioner can observe the eye region if required during the medical procedure to check on the status of the eye.

The adhesive used on the resiliently deformable base to attach the eye shield to the patient's face has a bond strength that is strong enough to maintain the eye shield in position on the patient's face, but not strong enough to damage the skin or pluck eyebrows on removal thereof. In one form the adhesive is a pressure-sensitive adhesive and may be a hypoallergenic adhesive that is designed to hold firmly onto the skin of said patient.

A suitable adhesive is preferably used to attach the cover plate to the upper surface of the base. The adhesive may be applied to the upper surface of the base or underside of the cover plate before joining.

The eye shield may be provided with different dimensions for use on a range of face shapes and sizes. In one form there may be child, adult small, adult medium, and adult large sizes.

In another aspect of the invention there is proposed a method of constructing the eye shield for releasable mounting to a face of a patient as claimed in claim 11.

### BRIEF DESCRIPTION OF THE DRAWINGS

The accompanying drawings, which are incorporated in and constitute a part of this specification, illustrate an implementation of the invention and, together with the description and claims, serve to explain the advantages and principles of the invention. In the drawings,
- Figure 1: is a front view of a perioperative eye shield of the present invention affixed to a patient's face, illustrating an orbital rim of the patient's face;
- Figure 2: is a front view of a second embodiment of the perioperative eye shield;
- Figure 3: is a front view of a third embodiment of the perioperative eye shield;
- Figure 4: is a front view of the perioperative eye shield of Figure 2;
- Figure 5: is a cross-sectional view through A-A of the perioperative eye shield of Figure 4;
- Figure 6: is a cross-sectional view through B-B of the perioperative eye shield of Figure 4;
- Figure 7: is a longitudinal cross-sectional view of the perioperative eye shield of Figure 4 illustrating the pivoting around the central hinge member;
- Figure 8: is the cross-sectional view of the perioperative eye shield of Figure 7 illustrating the flexing of the first and second eye portions of the cover plate;
- Figure 9: is a longitudinal cross-sectional view of a fourth embodiment of the perioperative eye shield illustrating an alternate hinge member, contoured base, grooves and ridges;
- Figure 10: is a front view of the perioperative eye shield of Figure 1 affixed to a patient's face, illustrating the attachment of an eye dressing used to hold the eyelids of a patient closed during surgery;
- Figure 11a: is a perspective exploded view of a fifth embodiment of the perioperative eye shield;
- Figure 11b: is a top view of the assembled eye shield of Figure 11a;
- Figure 11c: is a side view of the eye shield of Figure 11b;
- Figure 11d: is a front view of the eye shield of Figure 11b;
- Figure 12a: is a side view of the cover plate of Figure 11a illustrating the curvature along the lateral or vertical plane;
- Figure 12b: is a top view of the cover plate of Figure 11a illustrating the curvature along the longitudinal or horizontal plane;
- Figure 12c: is a front view of the cover plate of Figure 11a, illustrating the three holes that form the hinge member;
- Figure 13a: is a front view of the resiliently deformable base of Figure 11a, illustrating the apertures that align with the holes in the cover plate;
- Figure 13b: is a top view of the resiliently deformable base of Figure 13a before it is attached to the cover plate;
- Figure 14a: is a top view of the base of Figure 13a, illustrating its profile once it has been attached to the cover plate;
- Figure 14b: is a side view of the curved configuration of the base of Figure 14a; and
- Figure 14c: is a front view of the curved configuration of the base of Figure 14a.

### DETAILED DESCRIPTION OF THE ILLUSTRATED AND EXEMPLIFIED EMBODIMENTS

Similar reference characters indicate corresponding parts throughout the drawings. Dimensions of certain parts shown in the drawings may have been modified and/or exaggerated for the purposes of clarity or illustration.

Referring to the drawings for a more detailed description, an improved surgical eye shield 10 is illustrated, demonstrating by way of examples, arrangements in which the principles of the present invention may be employed. The figures illustrate an eye shield 10 for releasable mounting to a face 12 of a patient 14, used to inhibit pressure being applied to eyes 16, 18 of the patient 14 during a medical procedure.

The reader should understand that the term eye or eyes used throughout the specification, unless otherwise stated, relates to the eye region in general including the eyelids, eye globe and cornea. Furthermore, the term pressure should be understood to refer to the force per unit area applied down onto the surface of the eye shield by a person or object.

The eye shield 10 in one form is a perioperative eye shield and includes a resiliently deformable base 20, including apertures 22, 24 extending therethrough that correspond to the position of the eyes 16, 18 when the eye shield 10 is attached to the face 14. The base 20 includes an adhesive layer 26 on an underside 28 for attachment to the skin of the face 12 adjacent or overlaying the front of the orbital rims 30, illustrated by broken lines, of the patient 14. The orbit is the rigid bony cavity in the skull that contains the eyeball. The broken line generally delineates the anterior rim of the orbit.

A flexible, transparent cover plate 32 is attached to an upper surface of the base 20 and extends over the apertures 22, 24 thereby forming respective chambers 34 to protect the respective eyes 16, 18 from direct contact, when the eye shield 10 is attached to the face 12. The flexible cover plate 32 includes first and second eye cover portions 36, 38 and an integral hinge member 40 located therebetween.

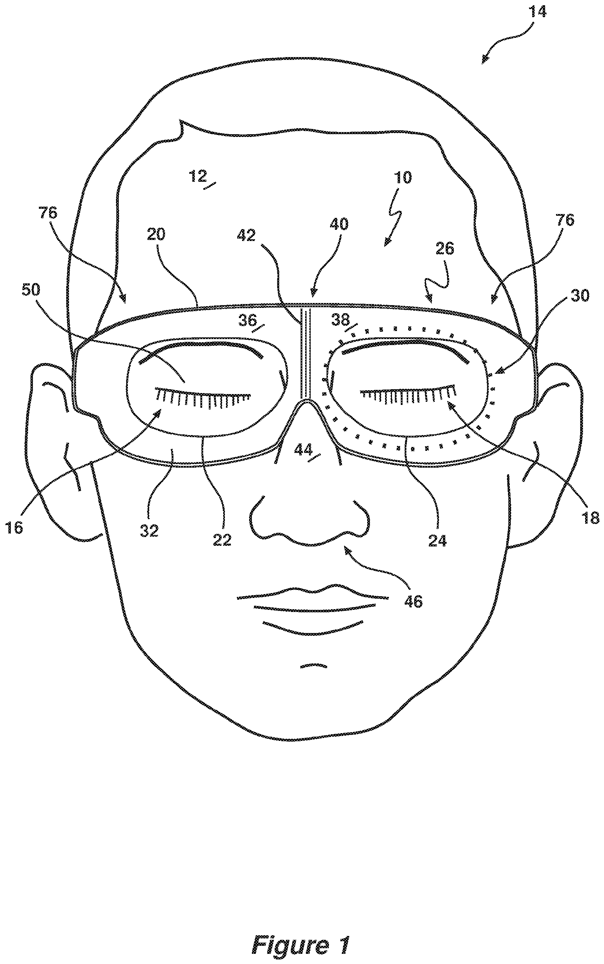

The first and second eye cover portions 36, 38 and the integral hinge member 40 are of unitary construction. As illustrated in Figure 1, the hinge member 40 may be formed by a line or lines of weakness 42 that extend down the cover plate 32 at a mid region thereof.

The reader should however appreciate that in an embodiment, which is not according to the present invention but which is useful for understanding the present invention, the transparent cover plate 32 may be constructed from two individual eye cover portions that are fixedly joined along adjacent edges by a hinge member wherein the hinge member overlays the bridge 44 of the patient's nose 46 when the eye shield 10 is attached to the face 12.

As further illustrated in Figure 1, the transparent cover plate 32 extends beyond the orbital rim 30 of the patient's face 12 such that when pressure is applied to a front face 48 of the transparent cover plate 32, the force is distributed around the bony orbital rims 30 and bridge 44 of the patient's nose 46 wherein the eye shield 10 is inhibited from impinging upon an eye or eyes 16, 18 of the patient 14.

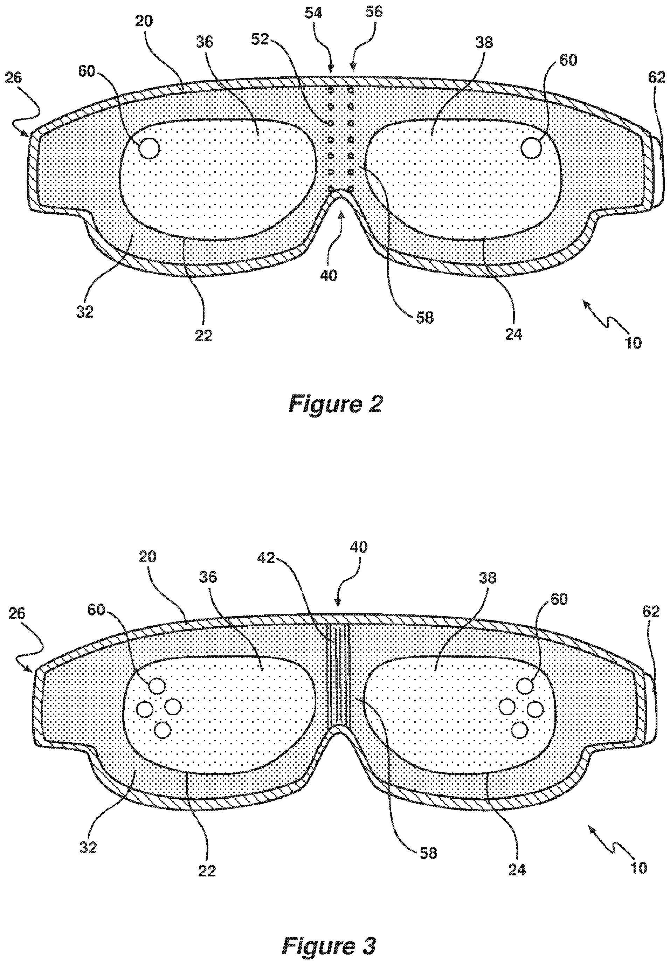

In another embodiment a plurality of holes 52 extend through the cover plate 32 to form a line of weakness thereby creating the hinge member 40. Figure 2 illustrated two lines 54 and 56 of holes 52, which form a region of weakness extending down the transparent cover plate 32.

The resiliently deformable base 20 may be constructed from medium density polyurethane foam, and includes an adhesive central part 58 that corresponds to the location of the hinge member 40. The adhesive central part 58 can be demountably attached to the bridge 44 of the patient's nose 46, to thereby create respective chambers 34, forward of eyes 16, 18 to protect them from direct contact, when the eye shield 10 is attached to the face 12.

The transparent cover plate 32 includes vent holes 60 extending therethrough to inhibit condensation within the chambers 34.

As further illustrated in Figure 2, the base 20 may include a non-adhesive tab 62 on one side of the shield 10 to aid in the application and removal of the eye shield 10. Although this is not essential and the practitioner can simply lift the edge of the base 20 off the skin to initiate the removal of the eye shield 10.

Figure 3 illustrates another embodiment wherein the hinge member 40 is formed by a plurality of parallel lines of weakness 42. Furthermore, the first and second eye cover portions 36, 38 may include a plurality of vent holes 60 extending therethrough.

As shown in Figures 2 and 3, the outer edge of the base 20 extends beyond the perimeter of the transparent cover plate 32, which means that when force is applied to the front 48 of the cover plate 32 the edge of the cover plate is inhibited from contacting the face 12. This is because as the base 20 is compressed it bulges outwardly, while at the same time the edge of the cover plate 32 curves inwardly through an arc as it is pushed towards the face 14.

It should be appreciated that in other embodiments the base 20 would not need to extend beyond the perimeter of the transparent cover plate 32, due to the unique properties of the material used to construct the base.

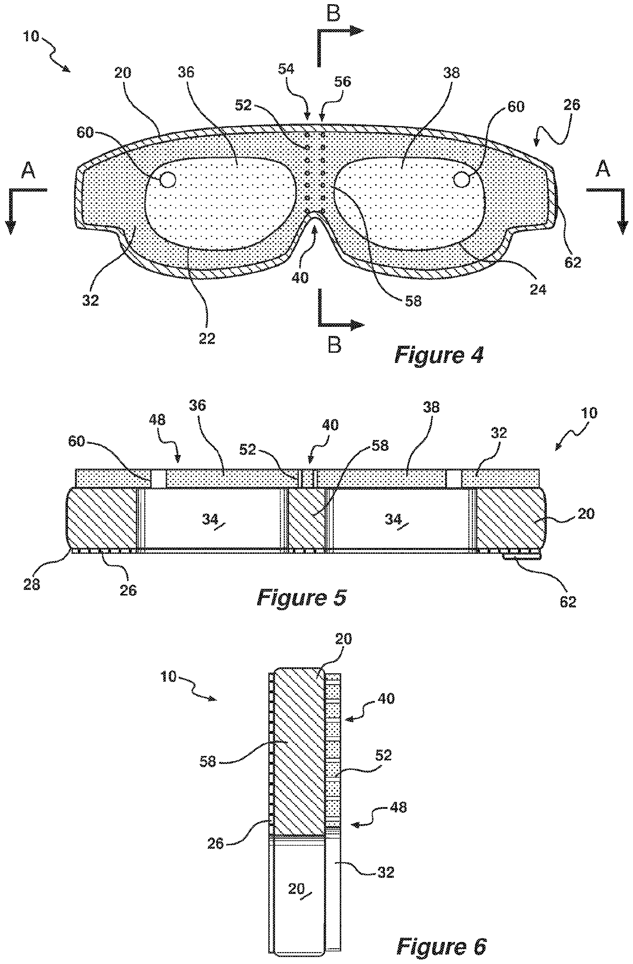

Figures 4 to 6 show different cross-sectional views through the face shield 10. As shows in Figure 5 the eye shield may include a non-peelable tab 62 that is attached to an underside of the adhesive layer 26.

As shown in Figures 5 and 6, the transparent cover plate 32, in one embodiment, is a generally thin, piece of material of uniform thickness. It should be appreciated that some of the dimensions such as the relative thicknesses have been exaggerated to illustrate the invention. The resiliently deformable base 20 may be of uniform thickness of between 10-12 mm, however it should be appreciated that the base is not limited to these dimensions.

The transparent cover plate 32 is semi-rigid to inhibit the eye shield 10 bowing inwardly and coming into contact with the eyes of the patient, however it is still flexible enough to permit flexing of the eye shield 10 around the contours of the face 12.

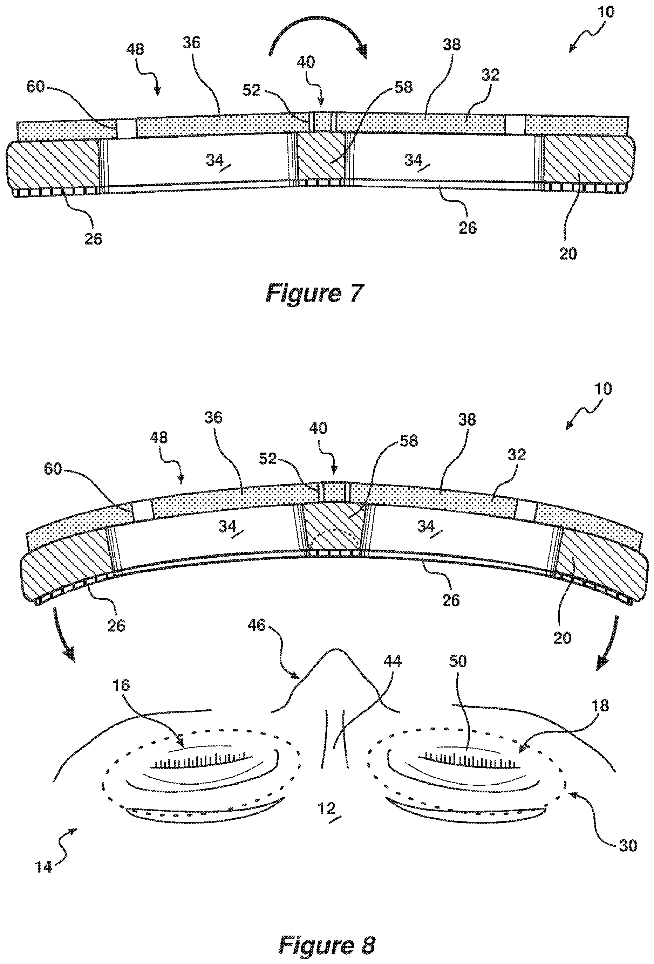

The reader will now appreciate that the transparent cover plate 32 is flexible enough to be bent or flexed along its width and height to conform to the face 12. The first and second eye cover portions 36, 38 are configured to pivot relative to each other about the hinge member 40, as illustrated by the arrow in Figure 7. The first and second eye cover portions 36, 38 are further configured to flex or bend along the width of the first and second eye covers 36, 38, as illustrated by the arrows in Figure 8, to generally conform to the contours of the face 12. Although not illustrated, the first and second eye covers 36, 38 are also configured to flex or bend along their height. This two stage ability to first pivot and then flex means that the transparent cover plate 32 can be constructed from material that is sufficiently strong to inhibit contact with the eye when pressure is applied to the eye shield 10 by inhibiting the eye cover from bowing inwardly and yet flexible enough to conform to the shape or contours of the face 12.

As further illustrated in Figure 8 the central part 58 of the resiliently deformable base 20 can compress to conform to the bridge of the nose, as indicated by the broken line.

The eye shield 10 is configured to conform closely to the contours of the face 12 and does not project excessively outwardly from the face 12 of the patient. Since the transparent cover plate 32 is able to firstly pivot and then flex, the thickness of the resilient deformable base can be kept to a minimum, which allows a low profile to ensue. This low profile means that the eye shield 10 is less likely to get in the way of a surgeon or other medical practitioner during a medical procedure.

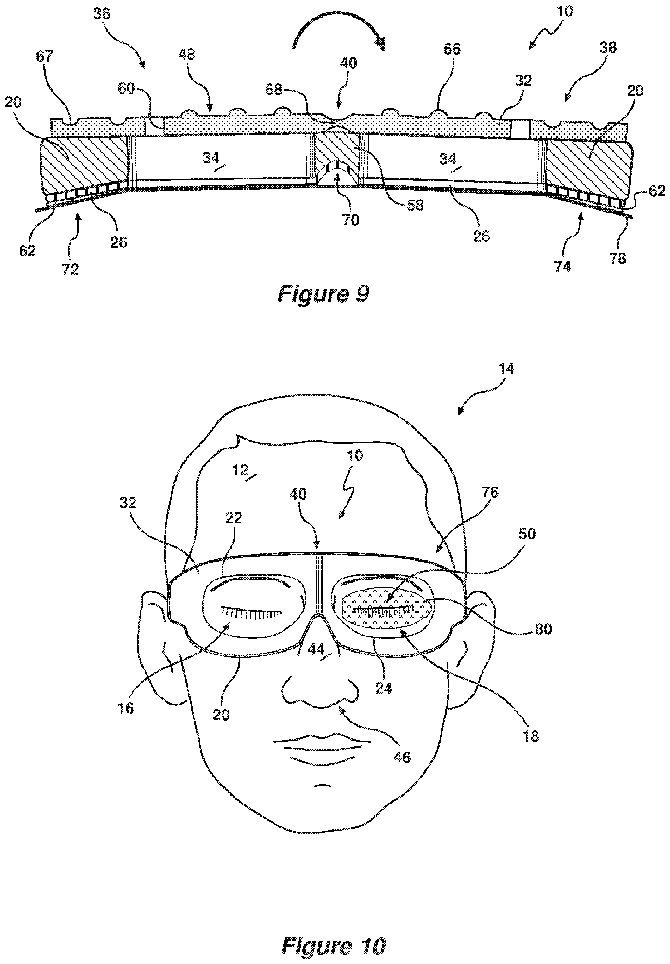

As illustrated in Figure 9, the transparent cover plate 32 can include ridges 66 that extend down the front of the cover plate 32. These ridges 66 can be used to increase the rigidity of the eye shield 10. The ridges 66 may be generally perpendicular to the longitudinal axis of the eye shield 10. Furthermore, grooves 67 may likewise extend down the front of the cover plate 32 to permit greater flexing at the edges of the transparent cover plate 32 adjacent the temples of the patient. It should be appreciated that a combination of grooves and ridges can be used.

The hinge member 40 in the present embodiment is a depression 68, formed by heating or pressing the cover plate 32. The depression 68 extends down the centre of the cover plate 32; wherein the first and second eye cover portions 36, 38 can pivot relative to each other.

The resiliently deformable base 20, of the embodiment of Figure 9, is moulded to facilitate attachment to the face. The central part 58 of the base 20 that is configured for attachment to the nose 46 of the patient 14 curves at 70 around a longitudinally extending axis to conform to the bridge 44 of the nose 46. As further illustrated in Figure 9 the outmost edges 72, 74 of the base 20 are tapered outwardly such that the outermost edge is thicker, to thereby facilitate attachment to the temples 76 of the patient 14.

In the present embodiment two non-peelable tabs 62 are attached to the underside of the adhesive layer 26 on either side of the base, as shown in Figure 9, so that the eye shield can be easily applied and removed from either side of the face, although it should be appreciate that such tabs are not essential.

Finally, as illustrated in Figure 9, a peelable protective film 78 extends over the adhesive layer 26 of the base 20 and non-peelable tabs 62, which protects the adhesive coating 26 prior to use. The peelable protective film 78 can be removed prior to the eye shield 10 being attached over the orbital rims of the face.

As shown in Figure 10, the eye shield 10 permits the attachment of an eye dressing 80 or tape. Such eye dressings 80 or tape are attached over the closed eyelids 50 of a patient 14 during surgery to inhibit the eyes from drying which may cause corneal damage. Since the cover plate 32 is transparent and the base 20 includes apertures 22, 24 the medical practitioner can observe the eye region if required during the medical procedure.

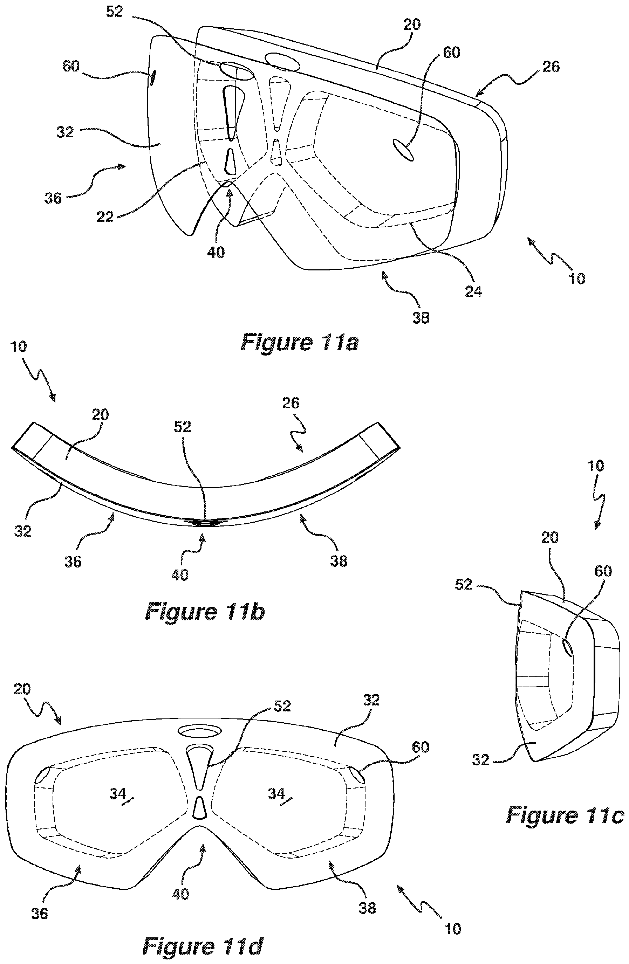

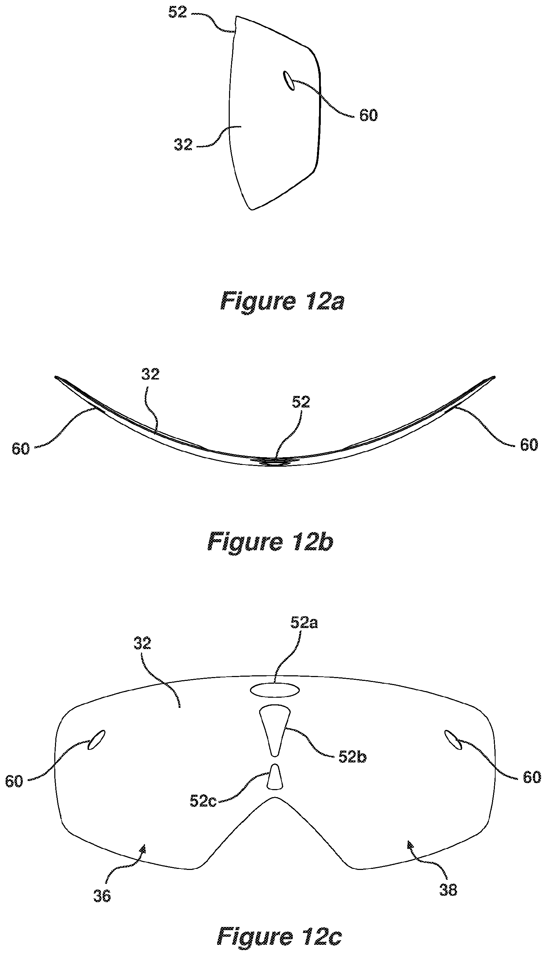

In another embodiment as illustrated in Figures 11a to 14c, each of the first and second eye cover portions 36, 38 of the eye shield 10 are curved along a line or plane extending longitudinally across the eye shield 10, as illustrated in Figures 11b and 12b. Furthermore the eye shield 10 is also curved along a line or plane extending laterally down the eye shield 10, as illustrated in Figures 11c and 12a. In this way the cover plate 32 and resiliently deformable base 20 attached thereto are curved in both vertical and horizontal directions.

The first and second eye cover portions 36, 38 of the eye shield 10 are able to pivot about the centrally located hinge member 40 and due to the material from which they are constructed, the first and second eye cover portions 36, 38 are able to flex to a lesser degree to conform to the contours of the face. The curvature of the first and second eye cover portions 36, 38 assists in maintaining contact between the eye shield 10 and the face 12 to inhibit it from peeling off during use. The curvature of the eye shield 10 along with the pivoting of the cover plate and flexing of the eye cover portions permit the eye shield to be used on various sized and shaped faces.

This is important in a hospital setting where the contours of the patient's face can vary due to the age of the patient and ethnicity.

The hinge member 40 in the present embodiment comprises holes 52a, 52b, 52c, that extend through the cover plate 32 at a mid region thereof, as illustrated in Figure 12c. The holes 52a, 52b, 52c, create generally speaking a line of weakness that extend laterally down the centre of the cover plate 32. The holes 52a, 52b, 52c, in the present embodiment are different shapes and sizes, however the reader should appreciate that the hinge member is not limited to the number, position or dimensions of the holes as illustrated.

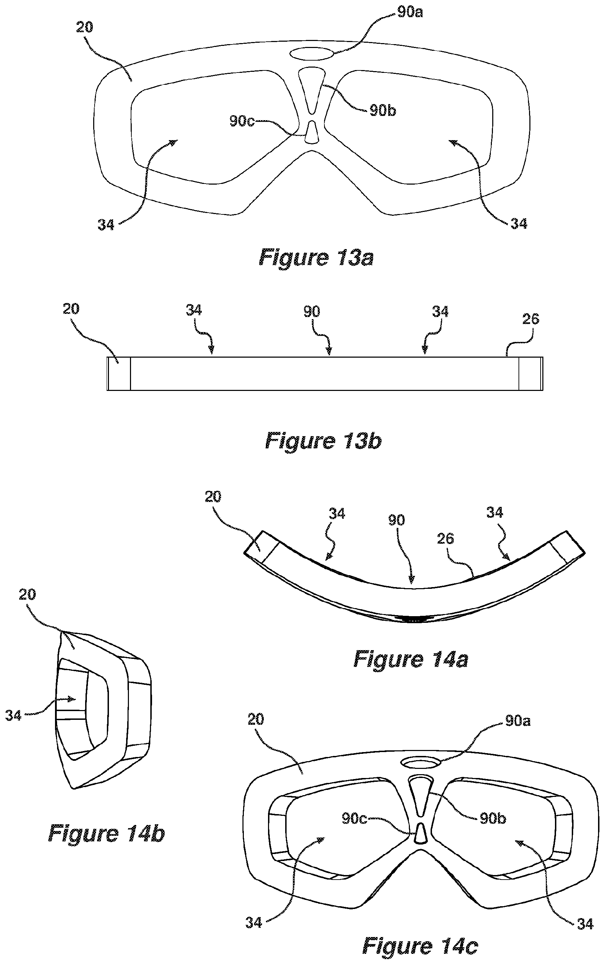

As further illustrated in Figure 13a, the base 20 includes holes 90a, 90b, 90c, that are of similar size to those in the cover plate 32 and which extend therethrough. The holes 90a, 90b, 90c, are configured to align with holes 52a, 52b, 52c, when the eye shield has been assembled, as illustrated in Figure 11d.

The base 20 in the present embodiment is cut from a flat sheet of medium density polyurethane foam, as illustrated in Figures 13a and 13b. The cover plate 32 is pre-curved or moulded such that it curves both longitudinally and laterally, as illustrated in Figures 12a, 12b. This means that when the base 20 is attached to the rear of the cover plate 32 it is likewise curved longitudinally and laterally, as illustrated in Figures 14a to 14c.

The invention also incorporates a method of constructing an eye shield 10 as disclosed above and a method of inhibiting pressure being applied to an eye or eyes of a patient during a medical procedure using the above eye shield.

The skilled addressee will now appreciate the advantages of the illustrated invention over the prior art. In one form the invention provides an eye shield that is a single use perioperative apparatus, which may be sterile, and covers both eyes of the patient to thereby inhibit pressure being applied to the eye or eyes. Since the transparent cover plate is constructed from a single piece, or two joined portions that form a single member to cover both eyes, when pressure is applied to one side of the shield the force is distributed across the orbital rims on both sides of the face and the bridge of the nose. This is important since it inhibits the edges of the cover plate from cutting into the edges of the eye as would occur with many of the devices suggested in the published prior art. This is accomplished by decreasing the force per unit area applied due to the distribution of the pressure since the device is a single unitary eye shield unit.

## Claims

1. An eye shield (10) for releasable mounting to a face (12) of a patient (14), used to inhibit direct pressure being applied to eyes (16, 18) of the patient (14) during a medical procedure, including;
a resiliently deformable base (20), including first and second apertures (22, 24) extending therethrough that correspond to a position of the eyes (16, 18) of the patient (14), the eye shield (10) being adapted to be attached to the face (12) around orbital rims (30) of the respective eyes (16, 18), the base (20) including an adhesive layer (26) on an underside (28) thereof, for attachment to the face (12), whereby the eye shield overlays both orbital rims of the patient;
**characterised by** a curved, flexible, transparent cover plate (32) attached to an upper surface of said base (20) and extending over said first and second apertures (22, 24), the cover plate (32) including first and second eye cover portions (36, 38) joined by an integral hinge member (40) located therebetween;
wherein the hinge member (40) has a greater degree of flex than the first and second eye cover portions (36, 38);
wherein the first and second eye cover portions (36, 38) are configured to pivot relative to each other around the hinge member (40) and flex along at least a part of their respective spans to generally conform to the shape of the face (12);
and wherein the first and second eye cover portions (36, 38) and integral hinge member (40) of the cover plate (32) are of unitary construction, the hinge member (40) extending down the cover plate (32), from a top to a bottom at a mid region thereof, the mid region corresponding to the position of a bridge (44) of the patient's nose (46) when the eye shield (10) is attached to the face (12) of the patient (14).

2. The eye shield (10) according to claim 1, wherein the integral hinge member (40) comprises a plurality of holes (52a, 52b, 52c) that extend therethrough.

3. The eye shield (10) according to claim 2, wherein the plurality of holes (52a, 52b, 52c) are uniform in size and shape, or the plurality of holes are dissimilar in size and shape.

4. The eye shield (10) according to claim 2 or 3, wherein the resiliently deformable base includes a plurality of holes (90a, 90b, 90c) that correspond in position, shape and size to the holes in the cover plate (32).

5. The eye shield (10) according to any one of the above claims, wherein the cover plate (32) is curved along a line extending longitudinally across the eye shield and is also curved along a line extending laterally down the eye shield.

6. The eye shield according to claim 1, wherein the transparent cover plate (32) extends beyond the orbital rims (30) of the patient's face (12) such that when pressure is applied to a front of the transparent cover plate (32) the force is distributed around the bony orbital rims and bridge (44) of the patient's nose (46), wherein the eye shield (10) is inhibited from impinging upon an eye of the patient (14).

7. The eye shield (10) according to any one of the above claims, first and second apertures (36, 38) create respective chambers (34) to protect the eyes (16, 18) from direct contact, when the eye shield (10) is attached to the face (12) of the patient (14).

8. The eye shield (10) according to any one of the above claims, wherein a peelable protective film (78) extends over the adhesive layer (26) of the base (20), which protects the adhesive layer prior to use and the base includes a non-adhesive tab (62) on at least one side of the shield (10) to aid in the application and removal of the eye shield.

9. The eye shield (10) according to any one of the above claims, wherein the transparent cover plate (32) includes vent holes (60) extending therethrough.

10. The eye shield (10) according to any one of the above claims, wherein the eye shield permits the attachment of an eye dressing or tape applied over closed eyelids of the patient to maintain the eyelids closed during surgery to inhibit corneal drying or abrasion, and the transparent cover plate (32) allowing a practitioner to observe the eye or eye dressings therethrough.

11. A method of constructing an eye shield (10) according to any one of claims 1 to 10 for releasable mounting to a face (12) of a patient (14), used to inhibit pressure being applied to eyes (16, 18) of the patient during a medical procedure, including the steps of:
selecting a curved sheet of flexible, transparent material (32) and forming a hinge member (40) extending down the transparent cover plate (32), from a top to a bottom at a mid region thereof, to thereby form the flexible cover plate (32);
selecting a resiliently deformable material and shaping or moulding a resiliently deformable base (20);
applying a first adhesive coating to the upper surface of said resiliently deformable base (20) and attaching the transparent cover plate (32) to the upper surface, or applying the first adhesive coating to the edge of an underside of the transparent cover plate (32), and attaching the transparent cover plate (32) to the upper surface of the resiliently deformable base (20);
applying a second adhesive coating (26) to an underside of the resiliently deformable base (20); and
overlaying the second adhesive coating (26) on the underside of the resiliently deformable base (20) with a non-adhesive peelable protective film (78) to inhibit damage prior to use.

12. The eye shield (10) according to any one of claims 1 to 10, wherein the hinge member (40) is formed by one or more lines of weakness (42) that extend down the cover plate (32) at a mid region thereof.

13. The eye shield (10) according to any one of claims 1 to 10, wherein the hinge member (40) is formed by a plurality of parallel lines of weakness (42).

14. The eye shield (10) according to claim 2, 3 or 4, the wherein the a plurality of holes (52a, 52b, 52c) of the hinge member (40) form a line of weakness.

15. The eye shield (10) according to claim 12, wherein the central part (58) of the resiliently deformable base (20) can compress to conform to the bridge (44) of the patient's nose (46).

## Patentansprüche

1. Augenschutz (10) zum lösbaren Anbringen an einem Gesicht (12) eines Patienten (14), der verwendet wird, um zu verhindern, dass während einer medizinischen Behandlung unmittelbarer Druck auf Augen (16, 18) des Patienten (14) ausgeübt wird, einschließend:
eine elastisch verformbare Basis (20), die eine erste und eine zweite Öffnung (22, 24), die sich durch dieselbe erstrecken, einschließt, die einer Position der Augen (16, 18) des Patienten (14) entsprechen, wobei der Augenschutz (10) angepasst ist, um an dem Gesicht (12) um Orbitalränder (30) der jeweiligen Augen (16, 18) befestigt zu werden, wobei die Basis (20) eine klebende Schicht (26) auf einer Unterseite (28) derselben, zur Befestigung an dem Gesicht (12), einschließt, wodurch der Augenschutz beide Orbitalränder des Patienten überdeckt,
**gekennzeichnet durch** eine gekrümmte, flexible, transparente Abdeckplatte (32), die an einer oberen Fläche der Basis (20) befestigt ist und sich über die erste und die zweite Öffnung (22, 24) erstreckt, wobei die Abdeckplatte (32) einen ersten und einen zweiten Augenabdeckabschnitt (36, 38) einschließt, die durch ein integrales Scharnierelement (40) verbunden sind, das zwischen denselben angeordnet ist,
wobei das Scharnierelement (40) einen größeren Biegungsgrad aufweist als der erste und der zweite Augenabdeckabschnitt (36, 38),
wobei der erste und der zweite Augenabdeckabschnitt (36, 38) dafür konfiguriert sind, im Verhältnis zueinander um das Scharnierelement (40) zu schwenken und sich entlang mindestens eines Teils ihrer jeweiligen Spannweiten zu biegen, um sich im Allgemeinen der Form des Gesichts (12) anzupassen,
und wobei der erste und der zweite Augenabdeckabschnitt (36, 38) und das integrale Scharnierelement (40) der Abdeckplatte (32) einen einheitlichen Aufbau haben, wobei sich das Scharnierelement (40) die Abdeckplatte (32) hinab, von einem Oberteil bis zu einem Unterteil an einem mittleren Bereich derselben, erstreckt, wobei der mittlere Bereich der Position einem Rücken (44) der Nase (46) des Patienten entspricht, wenn der Augenschutz (10) an dem Gesicht (12) des Patienten (14) befestigt ist.

2. Augenschutz (10) nach Anspruch 1, wobei das integrale Scharnierelement (40) eine Vielzahl von Löchern (52a, 52b, 52c) umfasst, die sich durch dasselbe erstrecken.

3. Augenschutz (10) nach Anspruch 2, wobei die Vielzahl von Löchern (52a, 52b, 52c) in Größe und Form gleichmäßig ist oder die Vielzahl von Löchern in Größe und Form unterschiedlich ist.

4. Augenschutz (10) nach Anspruch 2 oder 3, wobei die elastisch verformbare Basis eine Vielzahl von Löchern (90a, 90b, 90c) einschließt, die in Position, Form und Größe den Löchern in der Abdeckplatte (32) entsprechen.

5. Augenschutz (10) nach einem der vorhergehenden Ansprüche, wobei die Abdeckplatte (32) entlang einer Linie, die sich in Längsrichtung über den Augenschutz erstreckt, gekrümmt ist und ebenfalls entlang einer Linie, die sich in Seitenrichtung den Augenschutz hinab erstreckt, gekrümmt ist.

6. Augenschutz (10) nach Anspruch 1, wobei sich die transparente Abdeckplatte (32) derart über die Orbitalränder (30) des Gesichts (12) des Patienten hinaus erstreckt, dass, wenn Druck auf eine Vorderseite der transparenten Abdeckplatte (32) ausgeübt wird, die Kraft um die knöchernen Orbitalränder und den Rücken (44) der Nase (46) des Patienten verteilt wird, wobei verhindert wird, dass der Augenschutz (10) auf ein Auge des Patienten (14) einwirkt.

7. Augenschutz (10) nach einem der vorhergehenden Ansprüche, wobei die erste und die zweite Öffnung (22, 24) jeweilige Kammern (34) erzeugen, um die Augen (16, 18) vor unmittelbarer Berührung zu schützen, wenn der Augenschutz (10) an dem Gesicht (12) des Patienten (14) befestigt ist.

8. Augenschutz (10) nach einem der vorhergehenden Ansprüche, wobei sich ein abziehbarer schützender Film (78) über die klebende Schicht (26) der Basis (20) erstreckt, der vor der Verwendung die klebende Schicht schützt, und die Basis eine nicht-klebende Lasche (62) auf mindestens einer Seite des Schutzes (10) einschließt, um beim Anbringen und Entfernen des Augenschutzes zu unterstützen.

9. Augenschutz (10) nach einem der vorhergehenden Ansprüche, wobei die transparente Abdeckplatte (32) Lüftungslöcher (60) einschließt, die sich durch dieselbe erstrecken.

10. Augenschutz (10) nach einem der vorhergehenden Ansprüche, wobei der Augenschutz die Befestigung eines Augenverbandes oder Klebebandes ermöglicht, der/das über geschlossenen Lidern des Patienten angebracht wird, um die Augenlider während einer Operation geschlossen zu halten, um ein Austrocknen oder Abschürfen der Hornhaut zu verhindern, und die transparente Abdeckplatte (32) es einem Arzt ermöglicht, das Auge oder Augenverbände durch dieselbe zu beobachten.

11. Verfahren zum Aufbauen eines Augenschutzes (10) nach einem der Ansprüche 1 bis 10 zum lösbaren Anbringen an einem Gesicht (12) eines Patienten (14), verwendet, um zu verhindern, dass während einer medizinischen Behandlung unmittelbarer Druck auf Augen (16, 18) des Patienten ausgeübt wird, das die folgenden Schritte einschließt:
Auswählen einer gekrümmten Bahn eines flexiblen, transparenten Materials (32) und Bilden eines Scharnierelements (40), das sich die transparente Abdeckplatte (32) hinab, von einem Oberteil bis zu einem Unterteil an einem mittleren Bereich derselben, erstreckt, um dadurch die flexible Abdeckplatte (32) zu bilden,
Auswählen eines elastisch verformbaren Materials und Bilden oder Formen einer elastisch verformbaren Basis (20),
Aufbringen einer ersten klebenden Beschichtung auf die obere Fläche der elastisch verformbaren Basis (20) und Befestigen der transparenten Abdeckplatte (32) an der oberen Fläche oder Aufbringen der ersten klebenden Beschichtung auf die Kante einer Unterseite der elastisch verformbaren Basis (20) und Befestigen der transparenten Abdeckplatte (32) an der Oberseite der elastisch verformbaren Basis (20),
Aufbringen einer zweiten klebenden Beschichtung (26) auf eine Unterseite der elastisch verformbaren Basis (20) und
Überdecken der zweiten klebenden Beschichtung (26) auf der Unterseite der elastisch verformbaren Basis (20) mit einem nicht-klebenden abziehbaren schützenden Film (78), um eine Beschädigung vor der Verwendung zu verhindern.

12. Augenschutz (10) nach einem der Ansprüche 1 bis 10, wobei das Scharnierelement (40) durch eine oder mehrere Schwächungslinien (42) gebildet wird, die sich die Abdeckplatte (32) hinab an einem mittleren Bereich derselben erstrecken.

13. Augenschutz (10) nach einem der Ansprüche 1 bis 10, wobei das Scharnierelement (40) durch eine Vielzahl von parallelen Schwächungslinien (42) gebildet wird.

14. Augenschutz (10) nach Anspruch 2, 3 oder 4, wobei eine Vielzahl von Löchern (52a, 52b, 52c) des Scharnierelements (40) eine Schwächungslinie bildet.

15. Augenschutz (10) nach Anspruch 12, wobei sich der zentrale Teil (58) der elastisch verformbaren Basis (20) zusammendrücken kann, um sich an den Rücken (44) der Nase (46) des Patienten anzupassen.

## Revendications

1. Bouclier oculaire (10) destiné à être monté de manière amovible sur le visage (12) d'un patient (14), utilisé pour empêcher l'application d'une pression directe sur les yeux (16, 18) du patient (14) au cours d'une procédure médicale, incluant :
une base déformable élastiquement (20), incluant des première et deuxième ouvertures (22, 24) la traversant, qui correspondent à une position des yeux (16, 18) du patient (14), le bouclier oculaire (10) étant adapté pour être fixé sur le visage (12) autour des rebords orbitaux (30) des yeux respectifs (16, 18), la base (20) incluant une couche adhésive (26) sur un côté inférieur en vue d'une fixation sur le visage (12), le bouclier oculaire recouvrant ainsi les deux rebords orbitaux du patient ;
**caractérisé par** une plaque de couverture courbée, flexible, transparente (32) fixée sur une surface supérieure de ladite base (32) et s'étendant au-dessus desdites première et deuxième ouvertures (22, 24), la plaque de couverture (32) incluant des première et deuxième parties de couverture des yeux (36, 38) reliées par un élément de charnière (40) d'un seul tenant disposé entre elles ;
dans lequel l'élément de charnière (40) présente un degré de flexion supérieur aux première et deuxième parties de couverture des yeux (36, 38) ;
dans lequel les première et deuxième parties de couverture des yeux (36, 38) sont configurées pour pivoter l'une par rapport à l'autre autour de l'élément de charnière (40) et pour se fléchir le long d'au moins une partie de leurs extensions respectives pour s'adapter en général à la forme du visage (12) ;
et dans lequel les première et deuxième parties de couverture des yeux (36, 38) et l'élément de charnière (40) de la plaque de couverture (32) qui en fait partie intégrante ont une construction d'un seul tenant, l'élément de charnière (40) s'étendant vers le bas de la plaque de couverture (32), du haut vers le bas au niveau d'une région médiane de celle-ci, la région médiane correspondant à la position d'une arête (44) du nez (46) du patient lorsque le bouclier oculaire (10) est fixé sur le visage (12) du patient (14).

2. Bouclier oculaire (10) selon la revendication 1, dans lequel l'élément de charnière d'un seul tenant (40) comprend une pluralité de trous (52a, 52b, 52c) qui s'étendent à travers celui-ci.

3. Bouclier oculaire (10) selon la revendication 2, dans lequel la pluralité de trous (52a, 52b, 52c) ont une taille et une forme uniformes, ou la pluralité de trous ont une taille et une forme différentes.

4. Bouclier oculaire (10) selon les revendications 2 ou 3, dans lequel la base déformable élastiquement inclut une pluralité de trous (90a, 90b, 90c) qui correspondent en termes de position, de forme et de taille aux trous dans la plaque de couverture (32).

5. Bouclier oculaire (10) selon l'une quelconque des revendications précédentes, dans lequel la plaque de couverture (32) est courbée le long d'une ligne s'étendant longitudinalement à travers le bouclier oculaire et est également courbée le long d'une ligne s'étendant latéralement vers le bas du bouclier oculaire.

6. Bouclier oculaire selon la revendication 1, dans lequel la plaque de couverture transparente (32) s'étend au-delà des rebords orbitaux (30) du visage du patient (12) de telle sorte que, lors de l'application d'une pression sur une partie avant de la plaque de couverture transparente (32), la force est répartie autour des rebords orbitaux osseux et de l'arête (44) du nez (46) du patient, dans lequel le bouclier oculaire (19) est empêché de heurter un oeil du patient (14).

7. Bouclier oculaire (10) selon l'une quelconque des revendications précédentes, dans lequel des première et deuxième ouvertures (36, 38) créent des chambres respectives (34) pour protéger les yeux (16, 18) d'un contact direct, lorsque le bouclier oculaire (10) est fixé sur le visage (12) du patient (14).

8. Bouclier oculaire (10) selon l'une quelconque des revendications précédentes, dans lequel un film protecteur pelable (78) s'étend au-dessus de la couche adhésive (26) de la base (20) qui protège la couche adhésive avant l'utilisation, et la base inclut une languette non adhésive (62) sur au moins un côté du bouclier (10) pour faciliter l'application et le retrait du bouclier oculaire.

9. Bouclier oculaire (10) selon l'une quelconque des revendications précédentes, dans lequel la plaque de couverture transparente (32) inclut des trous d'aération (60) qui s'étendent à travers celle-ci.

10. Bouclier oculaire (10) selon l'une quelconque des revendications précédentes, dans lequel le bouclier oculaire permet la fixation d'un pansement oculaire ou d'une bande appliqué(e) au-dessus des paupières fermées du patient pour maintenir les paupières fermées au cours d'une intervention chirurgicale, pour empêcher le dessèchement ou l'abrasion de la cornée, et la plaque de couverture transparente (32) permettant à un praticien d'observer l'œil ou les pansements oculaires à travers celle-ci.

11. Procédé de construction d'un bouclier oculaire (10) selon l'une quelconque des revendications 1 à 10, destiné à être monté de manière amovible sur le visage (12) d'un patient (14), utilisé pour empêcher l'application d'une pression sur les yeux (16, 18) du patient au cours d'une procédure médicale, incluant les étapes suivantes :
sélection d'une feuille courbée de matériau flexible et transparent (32) et formation d'un élément de charnière (40) s'étendant vers le bas de la plaque de couverture transparente (32), du haut vers le bas au niveau d'une région médiane de celle-ci, pour former ainsi la plaque de couverture flexible (32) ;
sélection d'un matériau déformable élastiquement et formage ou moulage d'une base déformable élastiquement (20) ;
application d'un premier revêtement adhésif sur la surface supérieure de ladite base déformable élastiquement (20) et fixation de la plaque de couverture transparente (32) sur la surface supérieure ou application du premier revêtement adhésif sur le bord d'un côté inférieur de la paque de couverture transparente (32), et fixation de la plaque de couverture transparente (32) sur la surface supérieure de la base déformable élastiquement (20) ;
application d'un deuxième revêtement adhésif (26) sur un côté inférieur de la base déformable élastiquement (20) ; et
superposition du deuxième revêtement adhésif (26) sur le côté inférieur de la base déformable élastiquement (26) avec un film protecteur pelable non-adhésif (78) pour empêcher un endommagement avant l'utilisation.

12. Bouclier oculaire (10) selon l'une quelconque des revendications 1 à 10, dans lequel l'élément de charnière (40) est formé par une ou plusieurs lignes de faiblesse (42) qui s'étendent vers le bas de la plaque de couverture (32), au niveau d'une région médiane de celle-ci.

13. Bouclier oculaire (10) selon l'une quelconque des revendications 1 à 10, dans lequel l'élément de charnière (40) est formé par une pluralité de lignes de faiblesse parallèles (42).

14. Bouclier oculaire (10) selon les revendications 2, 3 ou 4, dans lequel une pluralité de trous (52a, 52b, 52c) de l'élément de charnière (40) forment une ligne de faiblesse.

15. Bouclier oculaire (10) selon la revendication 12, dans lequel la partie centrale (58) de la base déformable élastiquement (20) peut se comprimer pour s'adapter à l'arête (44) du nez (46) du patient.
